Europäisches Patentamt

(19) European Patent Office

Office européen des brevets

(11) Publication number: **0 067 622**
A1

# (12) EUROPEAN PATENT APPLICATION

(21) Application number: 82302842.8

(22) Date of filing: 02.06.82

(30) Priority: 04.06.81 GB 8117143

(43) Date of publication of application:
22.12.82 Bulletin 82/51

(84) Designated Contracting States:
AT BE CH DE FR GB IT LI LU NL SE

(51) Int. Cl.³: **A 61 F 13/02**
**A 61 L 15/06**

(71) Applicant: Simpson, John Michael
Smithy Cottage Hilton
Nr Blandford Dorset(GB)

(72) Inventor: Simpson, John Michael
Smithy Cottage Hilton
Nr Blandford Dorset(GB)

(74) Representative: Matthews, Howard Nicholas et al,
MATTHEWS HADDAN & CO HADDAN HOUSE 33
Elmfield Road
Bromley Kent BR1 1SU(GB)

(54) Surgical plaster.

㉗ The invention comprises a surgical plaster having a stretchable strip (10) carrying a pad (11), the pad being sufficiently incompressible to ensure that the pad will completely flatten and close a vein against escape of blood after a puncture of the vein or blood sampling.

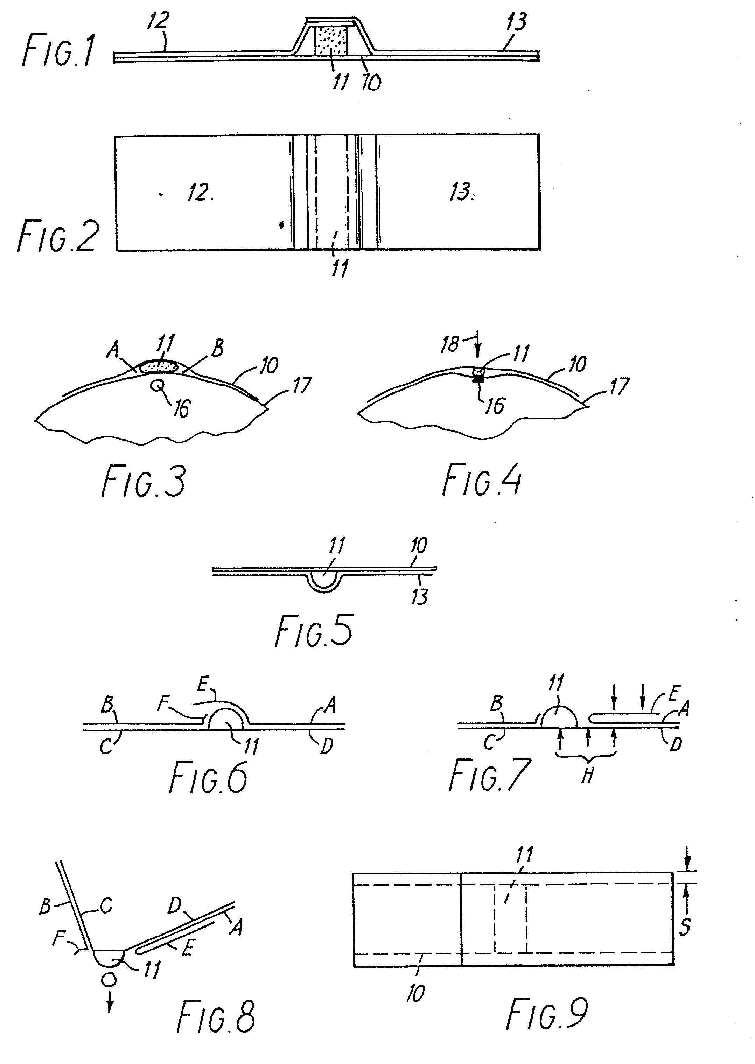

$FIG.1$

This invention relates to surgical plasters and its object is to prevent subcutaneous leakage of blood from vein punctures which may cause unsightly appearance (haematoma) resembling bruising such as occurs after intravenous injection or blood sampling and which is sometimes painful and may cause thrombosis of the vein and inability to use it for days or even weeks. This can be very important when very few veins are available for puncturing e.g. in intensive care or coronary care units where repeated sampling is necessary and anticoagulants are employed which make bleeding worse and can make it very difficult for the anaesthetist should an operation be necessary.

It is also important to reduce out-patient waiting time normally required to assess whether bleeding has stopped.

I have become aware that an attempt to meet this problem was made some years ago as described in United States patent specification No. 3490448 but this proved unsuccessful and was abandoned.

I am an anaesthetist and have made a careful study of this problem and have devised a form of plaster which gives completely successful results.

The plaster described in specification 3490448 was based on the idea of covering the skin puncture with an absorbent pad for two purposes viz (a) to absorb flow of blood and (b) engage the pad on the skin with sufficient pressure to minimize bleeding (not to stop it before it starts).

My invention is however based on a different principle viz to apply sufficient pressure directly on the vein to flatten it so as to prevent escape of blood from the vein, by anticipating the natural tendency of the skin to stretch after plaster application.

For this purpose I have found that the plaster must have the following distinctive features:

(1) The plaster must have an adhesive elastic strip stretchable with ease to at least 40 percent increase in length with strong tendency to assume its original length on release so as to draw the skin on both sides of the puncture together and press the pad on to the vein so as to completely flatten and close the vein - unlike 3490448 in which the strip is "preferably slightly elastic". A "slightly elastic" strip will effectively not flatten the vein after application.

The strip of the present invention will remain under tension when in position because the degree of stretch to which the plaster was subjected before application is greater than the skin stretchability. Thus as shown in Figure 3 of the accompanying drawings herewith the pad of 3490448 leaves gaps at A,B, with the adhesive strips leaving the skin at a large angle and showing that very little pressure remains after skin application whereas for the purpose of the present invention the pad must be substantially sunk into the flesh as shown in Figure 4 in order to flatten the vein.

(2) The strip should preferably be at least three inches in length in order to apply enough pressure to flatten the vein in all types of patients i.e. longer than in 3490448 which appears to be about two and a half inches.

(3) The elastic strip must be thin and preferably transparent because it is not sufficient to place the pad anywhere over the skin puncture as in 3490448, but the pad must be carefully positioned directly over the vein so the user must be able to see through the strip clearly.

(4) The strip must carry a pad which is not "compressible sponge material" as in 3490448 but on the contrary must be substantially incompressible in use e.g. less than 25 percent reduction in height under pressure of the adhesive strip or at any rate sufficiently incompressible to ensure permanent flattening and complete closure of the vein. It should require at least one pound weight to compress it 25 percent in height. I may be slightly absorbent but absorption is unimportant because blood does not leak from the flattened vein. The pad may be high density polyethylene foam.

(5) The pressure pad must not only be substantially incompressible in use but must be very narrow otherwise it is not possible to flatten the vein, as the wider the pad the less elastic recoil is left in the strip to overcome skin stretchability.

Thus the pad should be only 0.25 to 0.5 inch (.64 to 1.25cm) preferably 0.35 to 0.45 inch (.875cm to 1.125cm) wide which is very much less than the pad of 3490448.

(6) The height of the pad is also from 0.25 to 0.35 inch preferably 0.19 inch to 0.31 inch (0.5cm to 0.8cm) so that it sinks substantially into the flesh this being also required to enable the vein to be flattened. The pad may be of square cross-section.

(7) Preferably cover strips are provided over the adhesive side of the base strip and pad which are removed when the plaster is to be applied and these cover strips should have their inner ends overlapping one another directly over the pad.

(8) The strips and pad should be at least 2cms width wise of the strip.

(9) Preferably the pad is rounded on the side contacting the skin.

(10) The adhesive film may be a thin non fabric plastics film provided with innumerable perforations.

(11) The adhesive strip is preferably extensible to a length of at least 50 percent increase.

The invention will be further described by way of example with reference to the accompanying diagrammatic drawings wherein:-

FIGURE 1 is a side view of a plaster made in accordance with the invention;

FIGURE 2 is a plan view thereof;

FIGURE 3 shows the plaster applied to the wound as in Figure 11 of 3490448;

FIGURE 4 shows the plaster applied to the vein in accordance with the present invention; and

FIGURE 5 shows an alternative cross-sectional shape of pad in accordance with the invention; and

FIGURES 6 to 9 show modifications.

0067622

Figures 1 and 2 show a plaster consisting of a very thin transparent film 10 of elastically stretchable plastics material which is self-adhesive on its upper side. It is four inches long stretchable easily to six inches, alternatively it may be three inches long and stretchable to over four inches. Midway of its ends a solid block or pad 11 is fixed adhesively or by fusing or other means. The film on its adhesive side and the block 11 are covered by pieces of removable protective material 12,13 which overlap each other over the block 11. The block 11 is elongated across the film and is of square section with sides 0.7 to o.8mm.

The base film 10 should have a stretched length at least 140 (e.g. 150) percent of its normal length i.e. at least 40% more than its normal length. The block 11 should not be compressible in use more than 25 percent in height under the pressure of the adhesive strip.

In use, the protective material 12 is removed, the film 10 is stretched and applied to the skin 17 (Figure 4) with the block 11 on the vein 16. The film then tends to restore itself to normal length and compression results as indicated by the arrow 18 which flattens the vein.

It is important that the elastic deformation of the plaster should exceed that of the skin at the site of application, otherwise the compression forces will not act on the vein, but be transmitted totally to the skin.  Thus after application, as the plaster tends to restore itself to the unstretched state the natural stretch of the skin is first totally·absorbed, and yet enough elasticity will be left in the plaster for it to remain as a tense, rigid structure.

Compression of the vein occurs by:-

(I) The solid block maintains the deformation of the tense base film as the total elastic deformation of the base film exceeds the mobility of those parts of the skin to which the base film is adhesively attached the tense base film deformed by the said solid block still attempts to assume its own original length.  As the stretchability of the latter is greater than that of the skin and it lies on the skin as a rigid tense structure still attempting to assume its own original length of 4", and thus pushes the solid block away from it in the direction of least resistance i.e. downwards onto the vein.

(II) The tendency of the base film to assume its original length causes compression forces to act towards the block, and like an orange pip that is squeezed, the block is forced at right angles away from the film, i.e. onto the vein.

(III) The subcutaneous tissues are compressed onto the side walls of the vein due to the pull of the plaster on the skin to which the subcutaneous tissues are attached.

The plaster can also be applied by one person, is simple, and it does not matter whether one is right or left handed.

The plaster may be applied by stretching both ends simultaneously before application to the vein or by applying the solid and maintaining pressure on it by the thumb while stretching and applying first one side and then the other.

It can happen that a drop of blood can appear as the needle is withdrawn. However, this does not come from the vein but exudes from the needle and this also can be avoided if the needle is drawn out at a sufficiently large angle to the skin.

0067622

If desired a thin absorbent material may be provided over the block or wrapped around it but this is not deemed necessary.

The shape shown in Figure 5 combined with a small degree of compressibility assists in manufacture by automatic machinery and the rounded surface which contacts the skin aids in providing direct pressure on the vein.

In the arrangement shown in Figures 6 to 8 two protective coverings to the adhesive tape are made so that one (A) overlaps the pad 11 and has one margin which extends beyond it as at E whilst the other (B) has a much narrower margin F which rests on it. The margin E rests on the block 11 and extends over the narrow margin F.

The plaster is grasped by say the right hand (if left hand is controlling needle and syringe) with A and D being squeezed between thumb and forefinger.

The thumb would be tending to lie over the pad.

The middle finger of this right hand now reaches for flap E and pulls it back and compresses it against flap A so that now the flaps E,A and D are compressed between thumb, and index and middle fingers.  The bulk of the pad will make flap E stand up and make for easy access by the finger.

The pad is now pressed onto the needle by the thumb at H and the syringe withdrawn by the left (in this case) hand.

The flaps C and B are now raised up so that section F presents itself to view.

This is grasped, B F covering removed, C is stretched and applied to the skin.

The thumb exerting pressure on the pad is replaced by the other thumb, flap A E is removed and D is stretched and applied.

Section F should be long enough to be grasped but not so long that it will be compressed between pad and skin when thumb pressure is applied to the pad. If desired the protective coverings may be larger than the adhesive transparent plaster e.g. 2 to 3 cms wider on each side as at S.

This provides very satisfactory ease of grasping at removal time as the edge at its width can be gripped rather than the end at its length.

An alternative method of use is simply to strip the covering flange A E off before vein puncture is begun and to lay the plaster down, sterile side upwards of course.

When necessary it can be picked up by compression of B and C, the thumb pressing the pad down, the needle is removed, D is stretched and applied, and then B is stripped as above and stretch before application to the skin.

- 13 -                    0067622

The adhesive strip after being stretched in length should preferably be recoverable to reduce the increase to less than 20 percent increase in length.

The adhesive strip may extend on each side of the pad at least 3 cm. as at "S" Figure 9.

The strip may be sufficiently elastic to remain tensioned after application to the skin in at least a partly stretched state.

0067622

CLAIMS

1. A plaster adapted to compress a punctured vein to prevent completely escape of blood, having an adhesive strip and a pad carried by the strip at approximately midway between its ends, characterized in that said adhesive elastic strip (10) is stretchable by at least 40 percent of its length with strong tendency to recover towards its original length on release, and in that said elastic strip is sufficiently elastic to draw the skin together on both sides of the puncture to compress the pad on to the vein to flatten and close the vein, said pad being sufficiently incompressible to ensure complete flattening of the vein.

2. A plaster as claimed in claim 1, characterized in that the pad is 0.25 to 0.5 inch (0.64 to 1.25 cm) wide and 0.25 to 0.35 inch (0.64 to 0.875 cm) in height.

3. A plaster as claimed in claim 1, chacterized in that the pad is 0.35 to 0.45 inch (0.875 to 1.125 cm) wide and 0.19 to 0.31 inch (0.5 to 0.8 cm) in height.

4. A plaster as claimed in claim 1, characterized in that the pad is rounded on its application side.

5. A plaster as claimed in claim 1 or 2, having removable cover strips (12, 13) on the adhesive.

6. A plaster as claimed in claim 1, characterized in that two cover strips (12, 13) are provided, on the adhesive strip, one of which has a narrow margin (F) resting on the pad and the other has a larger marginal part (E) which rests on the pad and extends over the said narrow margin.

7. A plaster as claimed in claim 5 or 6, characterized in that the cover strips are wider than the adhesive strip.

8. A plaster as claimed in any of the preceding claims, characterized in that the elastic strip is transparent.

9. A plaster as claimed in any of the preceding claims, characterized in that the elastic strip is a thin non-fabric transparent plastics film provided with many perforations.

10. A plaster as claimed in any of the preceding claims, characterized in that the pad is not compressible more than 25% of its height by the elastic strip.

11. A plaster as claimed in any of the preceding claims, characterized in that the pad has a substantially non-absorbent surface.

12. A plaster as claimed in any of the preceding claims, characterized in that the adhesive strip is stretchable by at least 50 percent of its length, and recovers to less than 20 percent.

13. A plaster as claimed in any of the preceding claims, characterized in that the adhesive strip extends on each side of the pad at least 3 cm.

14. A plaster as claimed in any of the preceding claims, characterized in that the adhesive strip and pad are at least 2cm. widthwise of the strip and the pad is 0.64 to 1.25 cm. lengthwise of the strip.

15. A plaster as claimed in any of the preceding claims, characterized in that the pad requires a weight of at least one pound (2.24 Kg.) to compress it 25 percent in height.

16. A plaster as claimed in any of the preceding claims, characterized in that the adhesive strip is sufficiently elastic to remain tensioned after application to the skin in at least a partly stretched state.

0067622

FIG.1

12       13

11 10

FIG.2

12.     13:

11

FIG.3

A   11   B

10

17

16

FIG.4

18

11   10

16   17

FIG.5

11    10

13

FIG.6

E

F

B      A

C     11     D

FIG.7

11

E

B      A

C       D

H

FIG.8

B   C

D

F     E   A

11

FIG.9

11

10

S

## European Patent Office

## EUROPEAN SEARCH REPORT

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| A,D | US - A - 3 490 448 (GRUBB) <br> * Totality * | | A 61 F 13/02 <br> A 61 L 15/06 |
| A | GB - A - 1 406 990 (JOHNSON) <br> * Claims 1,4 * | | |
| A | GB - A - 1 309 768 (JOHNSON) <br> * Claim 1; page 1, lines 55-58 * | | |

**TECHNICAL FIELDS SEARCHED (Int.Cl. ³)**

A 61 F

A 61 L

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document. but published on, or after the filing date
D: document cited in the application
L: document cited for other reasons

&: member of the same patent family, corresponding document

| X | The present search report has been drawn up for all claims | | |
|---|---|---|---|
| Place of search <br> VIENNA | Date of completion of the search <br> 05-08-1982 | Examiner <br> FARNIOK | |

EPO Form 1503.1 06.78